# EUROPEAN PATENT APPLICATION

(11) **EP 2 266 945 A1**
(43) Date of publication of application: **29.12.2010**
(21) Application number: 10172824.4
(22) Date of filing: 01.02.2008
(51) Int. Cl.: C07C 67/26, C07C 69/54, B01D 5/00

(54) **A process for the production of hydroxyalkyl (meth)acrylates**

(30) Priority: 14.02.2007 US 901573 P
(62) Divisional of application: 08250398.8
(71) Applicant: Rohm and Haas Company, Philadelphia, Pennsylvania 19106-2399 (US)
(72) Inventor: Curtis, Michael, A, Houston, TX 77005 (US)
(74) Representative: Kent, Venetia Katherine

(57) **Abstract**

The present invention relates to a process for producing high purity hydroxyalkyl (meth)acrylates. Specifically, the present invention relates to an improved process which can commercially and advantageously yield high purity hydroxyalkyl (meth)acrylates from (meth)acrylic acid and alkylene oxides.

## Description

The present invention relates to a process for producing hydroxyalkyl (meth)acrylates. Specifically, the present invention relates to an improved process which can commercially and advantageously yield hydroxyalkyl (meth)acrylates from (meth)acrylic acid and alkylene oxides.

As used herein, the use of the term "(meth)" followed by another term such as acrylate refers to both acrylates and methacrylates. For example, the term "(meth)acrylate" refers to either acrylate or methacrylate; the term "(meth)acrylic" refers to either acrylic or methacrylic; the term "(meth)acrylic acid" refers to either acrylic acid or methacrylic acid.

Achieving high degrees of purity, defined as purity greater than 98%, is one problem that exists in the process of producing hydroxyalkyl (meth)acrylates. Attempts to achieve high degrees of purity hydroxyalkyl (meth)acrylates as exemplified in US 4,365,081 A and US 6,465,681 are produced by reacting (meth)acrylic acid with an alkylene oxide in the presence of a homogenous catalyst. Processes such as the one disclosed in US 4,365,081 A are traditionally conducted under specific process conditions such as temperature and feed rate. Typically, the alkylene oxides are fed into the reactor at rates below 0.27 moles of alkylene oxide per mole of (meth)acrylic acid per hour, with reaction temperatures held constant and above 69 °C. Purity levels of only around 97.5% are achieved. Similarly, the process disclosed in US 6,465,681 was conducted under specific feed rate and temperature conditions, wherein the alkylene oxides were fed into the reactor at rates ranging from 0.71 to 0.18 and the reaction temperature was increased after 50% conversion of (meth)acrylic acid and exceeded 69 °C. With process conditions of this nature, one experiences purity levels of the resulting reaction liquid of only 91.4%. Increasing levels of product purity while balancing economic and process efficiency continue to be goals of hydroxyalkyl (meth)acrylate producers.

The present invention has solved this problem by increasing purity levels to greater than 98% through optimizing the process conditions of temperature and feed rate. As a result of the optimization, the present invention demonstrates lower byproduct formation, increased performance in downstream applications, and more uniformity of resultant polymers when these monomers are used in production over the monomers of the prior art as aforementioned.

In a first aspect of the present invention, there is provided a process for producing a high purity hydroxyalkyl (meth)acrylate comprising:
i) feeding (meth)acrylic acid into a reaction vessel;
ii) feeding at least one alkylene oxide into the reaction vessel at a feed rate in the range of 0.12 to 1.23 moles of alkylene oxide per mole of (meth)acrylic acid per hour;
iii) maintaining a reaction temperature during the supply of the alkylene oxide in the range of 50-69 °C; thereafter
iv) cooling the reactor contents to a temperature lower than the initial reaction temperature by at least 5 °C wherein cooling is initiated when at least 35 wt% of the total amount of alkylene oxide to be added has been added to the reactor;
v) reacting the at least one (meth)acrylic acid and at least one alkylene oxide to form a hydroxyalkyl (meth)acrylate wherein the hydroxyalkyl (meth)acrylate comprise impurities; and
vi) purifying the hydroxyalkyl (meth)acrylate via distillation.

In a second aspect of the current invention there is provided a process for producing a high purity hydroxyalkyl (meth)acrylate comprising:
i) feeding (meth)acrylic acid into a reaction vessel;
ii) feeding at least one alkylene oxide into the reaction vessel at a feed rate in the range of 0.12 to 1.23 mole of alkylene oxide per mole of (meth)acrylic acid per hour;
iii) reacting the at least one acrylic acid and at least one alkylene oxide to form a hydroxyalkyl (meth)acrylate;
iv) purifying the hydroxyalkyl (meth)acrylate via distillation
v) cleaning the reaction vessel with at least one wash wherein the components of the wash consist essentially of acetic acid and water.

(Meth)acrylic acid is fed into a reaction vessel. At least one alkylene oxide is fed into the same reactor at predetermined feed rates. Suitable feed rates for the alkaylene oxide range from upper limits of 1.23, 1.20, and 1.17 to lower limits of 0.12, 0.20, and 0.26. All ranges are inclusive and combinable.

The reaction is conducted using equipment and basic process that is well known by those of skill in the art. US 4,365,081 A, exemplifies the basic equipment for use in the process of the present invention.

Reactants are reacted in the presence of a catalyst to form at least one hydroxyalkyl (meth)acrylate and impurities. Examples of high purity hydroxyalkyl (meth)acrylates produced by the present invention include but are not limited to hydroxyethyl acrylate, hydroxyethyl methacrylate, hydroxypropyl acrylate, and hydroxypropyl methacrylate. While the reactants are fed to the reactor the temperature of the vessel is less than 69 °C, particularly in the range of 50 to 69 °C.

Subsequently, the reaction is performed at temperatures ranging from to upper limits of 100, 88, and 75 °C to lower limits of 25, 37, and 50 °C. All temperature ranges are inclusive and combinable.

After performing the reaction, the hydroxyalkyl (meth)acrylate product is then purified. In one aspect, the hydroxyalkyl (meth)acrylate product is distilled from the crude reaction mixture. One of the major problems with the distillation process is that the hydroxyalkyl (meth)acrylate itself is capable of further esterification with an additional (meth)acrylic acid molecule or transesterification with another hydroxyalkyl (meth)acrylate to produce an alkylene glycol di(meth)acrylate (hereinafter referred to as "di(meth)acrylate"). The di(meth)acrylate is undesirable because it can act as a crosslinking agent in any subsequent polymerizations in which the hydroxyalkyl (meth)acrylate is a component. Furthermore, the di(meth)acrylate may increase the extent of unwanted polymerization during the distillation step resulting in increased amounts of tars or other unwanted solids in the still bottoms or on the equipment and a corresponding reduction in the yield of the desired hydroxyalkyl (meth)acrylate in the distillate. The extent of formation of this di(meth)acrylate is dependent on the type of catalyst used in the reaction, the concentration of the various reactants, products, or other additives, as well as the reaction and distillation conditions.

To combat this problem of increased amounts of tars or other unwanted solids, one or more diester retarders are customarily added to the reaction vessel. With specific hydroxyalkyl (meth)acrylates such as hydroxyethyl methacrylate (HEMA), hydroxypropyl acrylate (HPA), and hydroxypropyl methacrylate (HPMA) it is advantageous to exclude the use of diester retarders such as salicylic acid and other carboxylic acids or other acids as disclosed for this purpose in US 5,342,487 A when using a chromium catalyst. Typically these acids are used as diester retarders because they slow the formation of di(meth)acrylates. In addition, they are known to behave as thinning agents for distillation residue which is oftentimes very viscous. The problem with using these diester retarders is that they react with the components of the reaction mixture to form (meth)acrylic acid. (Meth)acrylic acid is an undesirable product in the reaction vessel because it is difficult to separate from the desired hydroxyalkyl (meth)acrylate. Therefore it is necessary to manipulate process conditions to control diester formation without using diester retarders such as carboxylic acids for hydroxyethyl methacrylate, hydroxypropyl acrylate, and hydroxypropyl methacrylate.

According to the present invention, chromium acetate and specific reactor and distillation conditions keep di(meth)acrylate levels low. This is advantageous because a conventional thinning agent or diester retarder as described above is not required. Additionally, decreased reaction temperatures result in decreased diester formation. By decreased reaction temperatures, it is meant temperatures ranging from 45 to 69 °C, more particularly from 50 to 69 °C.

Furthermore, it has been determined that increasing the feed rate of the oxide results in faster cycle times, lower byproduct formation, and increased product purity. Suitable increased feed rates range from upper limits of 0.61 to lower limits of 0.22.

A carrier may be added during the distillation of the hydroxyalkyl (meth)acrylate product. Suitable carriers of the present invention are comprised of hydroxyalkyl (meth)acrylates and small amounts of water. Any carriers may be used separately or in combination.

A wash may be added to the reaction vessel after the reaction. The wash is comprised solely of water and acetic acid provided in ratios ranging from upper limits of 100% water and 0% acetic acid to lower limits of 5% water and 95% acetic acid. All ratios are inclusive and combinable. The water and acetic acid may be added concurrently or stepwise and a wash process may consist of one wash or multiple washes. When adding the wash, it is preferable that the wash have a temperature that ranges from upper limits of 75, 55, and 40 °C, to lower limits of 25, 30, and 35 °C. All temperature ranges are inclusive and combinable.

### EXAMPLES

Hereinafter, examples and comparative examples of the present invention are more specifically illustrated. However the present invention is not limited to the following examples.

### Example 1

A stainless steel-316 (SS-316) reactor equipped with stirrer, heating coils and a heat exchanger was filled to 64 percent of capacity with acrylic acid, iron(0) powder equal to 0.56 percent of the initial acrylic acid charge by weight, and a second acrylic acid charge containing 4.6 weight percent of *p*-methoxyphenol (as polymerization inhibitor), and 3.0 weight percent of copper dibutyldithiocarbamate, (as polymerization inhibitor) equal to 4.6 percent of the initial acrylic acid charge by weight. The atmosphere was then replaced with air and nitrogen to achieve an atmosphere containing 9 percent oxygen. The temperature was raised to 69 °C and the mixture was stirred for 1.3 hours to produce iron(III) acrylate. During this period the oxygen concentration in the head space was maintained between 2.5 and 9 percent. Next the temperature was lowered to 68 °C and kept constant while ethylene oxide was fed at a rate of 0.22 moles of ethylene oxide per mole of acrylic acid per hour. After 35 percent of the total amount of ethylene oxide to be added was fed, the temperature was lowered and kept constant at 56 °C for the remainder of the ethylene oxide feed. The feed of ethylene oxide was ceased when the molar ratio of ethylene oxide to total acrylic acid reached 1.1 to 1, respectively. Next the temperature was kept constant at 56 °C to continue the reaction until the amount of unreacted acrylic acid decreased to less than 0.75 percent. The total reaction time was 9.1 hours. A gas-chromatographic analysis of the resultant mixture showed a concentration of hydroxyethyl acrylate of 91.8 weight percent, diethylene glycol monoacrylate of 7.2 weight percent, ethylene glycol diacrylate of 0.22 weight percent, and ethylene glycol of 0.05 weight percent. These results are shown in Table 1.

### Comparative Example 1

A procedure was performed employing the process of Example 1 except the ethylene oxide was fed at a rate of 0.18 moles of ethylene oxide per mole of acrylic acid per hour. The total reaction time was 10.4 hours, and a gas-chromatographic analysis of the resultant mixture showed a concentration of hydroxyethyl acrylate of 91.3 weight percent, diethylene glycol monoacrylate of 7.5 weight percent, ethylene glycol diacrylate of 0.17 weight percent, and ethylene glycol of 0.05 weight percent. These results are shown in Table 1.

**Table 1**

| | EO feed rate (moles EO/mole acrylic acid x h) | Initial Reaction Temp (°C) | Reaction Temp after 35% EO fed (°C) | HEA (wt%) | DEGMA (wt%) | EGDA (wt%) | EG (wt%) | Reaction Time (h) |
|---|---|---|---|---|---|---|---|---|
| Example 1 | 0.22 | 68 | 56 | 91.8 | 7.2 | 0.22 | 0.05 | 9.1 |
| Comparative Example 1 | 0.18 | 68 | 56 | 91.3 | 7.5 | 0.17 | 0.05 | 10.4 |

| | | | | | | | | |
|---|---|---|---|---|---|---|---|---|
| EO = Ethylene oxide HEA = Hydroxyethyl acrylate DEGMA = Diethylene glycol monoacrylate EGDA = Ethylene glycol diacrylate EG = Ethylene glycol | | | | | | | | |

### Example 2

A SS-316 reactor equipped with stirrer, heating coils and a heat exchanger was filled to 56 percent of capacity with acrylic acid, chromium(III) acetate powder equal to 0.75 percent of the initial acrylic acid charge by weight, and a second acrylic acid charge containing 4.9 weight percent of *p*-methoxyphenol (as polymerization inhibitor), and 3.1 weight percent of copper dibutyldithiocarbamate, (as polymerization inhibitor) equal to 3.9 percent of the initial acrylic acid charge by weight. The atmosphere was then replaced with air and nitrogen to achieve an atmosphere containing 9 percent oxygen. Next the temperature was raised to 65 °C and kept constant while propylene oxide was fed at a rate of 0.36 moles of propylene oxide per mole of acrylic acid per hour. The feed of propylene oxide was ceased when the molar ratio of propylene oxide to total acrylic acid reached 1.08 to 1, respectively. Next the temperature was lowered to 60 °C, and the temperature was kept constant at 60 °C to continue the reaction until the amount of unreacted acrylic acid decreased to less than 0.08 percent. The total reaction time was 9.0 hours. Next, the resultant mixture was transferred at a pressure of less than or equal to 8.0 kPa to a SS-316 distillation apparatus equipped with distillation vessel, demister pads, reboiler, contact condenser, and receiver tanks to carry out purification by distillation under vacuum of 0.0013 to 0.27 kPa with a mixture temperature range of 58 to 66 °C. Prior to transfer, the distillation vessel was charged with hydroxypropyl acrylate containing 6.5 weight percent of copper dibutyldithiocarbamate, (as polymerization inhibitor) equal to 2.9 percent of the initial acrylic acid charge by weight and a second hydroxypropyl acrylate charge containing 5.0 weight percent of *p*-methoxyphenol (as polymerization inhibitor), equal to 4.7 percent of the initial acrylic acid charge by weight. The transferred mixture was then held for 3 hours at 56 °C under a dynamic vacuum of 0.80 kPa to strip excess propylene oxide. During the strip phase air was introduced prior to the reboiler at a rate of 1.7*10⁻³ liters per minute per liter of distillation vessel volume. Next the material was distilled at a vacuum of less than 0.07 kPa with a mixture temperature range of 58 to 66 °C. During the distillation phase, air at a rate of 1.7*10⁻³ liters per minute per liter of distillation vessel volume was added prior to the reboiler, and air was added at a rate of 4.3*10⁻⁴ liters per minute per liter of distillation vessel volume to the contact condenser loop. Also during the distillation phase, recycled hydroxypropyl acrylate equal to 51 percent of the initial acrylic acid charge by weight was added to the crude mixture contained in the distillation vessel. The amount of commercial quality hydroxypropyl acrylate obtained was 193 percent relative to the initial amount of acrylic acid charged to the reactor. An additional amount of hydroxypropyl acrylate in the amount of 20 weight percent relative to the initial amount of acrylic acid charged to the reactor was also collected. A gas-chromatographic analysis of the resultant commercial mixture showed a concentration of hydroxypropyl acrylate of 98.4 weight percent, dipropylene glycol monoacrylate of 0.90 weight percent, propylene glycol diacrylate of 0.12 weight percent, and propylene glycol of 0.08 weight percent. These results are shown in Table 2.

### Comparative Example 2

A procedure was carried out in the same way as of Example 2 except salicylic acid in the amount equal to 0.35 percent of the initial acrylic acid charge by weight was added to the distillation vessel prior to the start of the distillation step. A gas-chromatographic analysis of the resultant commercial mixture showed a concentration of hydroxypropyl acrylate of 98.4 weight percent, dipropylene glycol monoacrylate of 0.84 weight percent, propylene glycol diacrylate content of 0.13 weight percent, and propylene glycol of 0.07 weight percent. These results are shown in Table 2.

**Table 2**

| | Salicylic Acid (Weight % of initial acid charged to reactor) | HPA (wt%) | DPGMA (wt%) | PGDA (wt%) | PG (wt%) |
|---|---|---|---|---|---|
| Example 2 | 0 | 98.4 | 0.90 | 0.12 | 0.08 |
| Comparative Example 2 | 0.35 | 98.4 | 0.84 | 0.13 | 0.07 |

| | | | | | |
|---|---|---|---|---|---|
| HPA = Hydroxypropyl acrylate DPGMA = Dipropylene glycol monoacrylate PGDA = Propylene glycol diacrylate PG = Propylene glycol | | | | | |

### Example 3

A 250 ml Erlenmeyer flask was charged with 28 grams of water, 62 grams of hydroxyethyl acrylate, and 10 grams of the ammonium salt of N-nitrosophenylhydroxylamine. The mixture was stirred for two hours during which the solution became homogenous. By weight, the homogenous solution had a ratio of the ammonium salt of N-nitrosophenylhydroxylamine to water to hydroxyethyl acrylate of 1: 2.8: 6.2.

### Comparative Example 3

A 250 ml Erlenmeyer flask was charged with twenty grams of the ammonium salt of N-nitrosophenylhydroxylamine and 90 grams of water. The mixture was stirred for two hours and then filtered. The composition of the filtrate was determined to be 9 parts water to 1 part of the ammonium salt of N-nitrosophenylhydroxylamine. Simultaneously a 250 ml Erlenmeyer flask was charged with twenty grams of the ammonium salt of N-nitrosophenylhydroxylamine and 90 grams of hydroxyethyl acrylate. The mixture was stirred for two hours and then filtered. Analysis by weight showed no appreciable concentration of the ammonium salt of N-nitrosophenylhydroxylamine in hydroxyethyl acrylate. Next the water solution and hydroxyethyl solution were mixed in ratio of 1 : 2 respectively. By weight the final homogenous solution had a ratio of the ammonium salt of N-nitrosophenylhydroxylamine to water to hydroxyethyl acrylate of 1 : 9 : 20.

### Example 4

A procedure would be carried out in the same way as Example 1 except the crude material would be purified by distillation in the following manner. The crude reaction mixture would be transferred at a pressure of less than or equal to 8.0 kPa to a SS-316 distillation apparatus equipped with distillation vessel, demister pads, reboiler, contact condenser, and receiver tanks to carry out purification by distillation under vacuum of 0.0013 to 0.27 kPa with a mixture temperature range of 58 to 73 °C. Prior to transfer, the distillation vessel would be charged with hydroxyethyl acrylate containing 6.1 weight percent of copper dibutyldithiocarbamate, (as polymerization inhibitor) equal to 4.0 percent of the initial acrylic acid charge by weight and a second hydroxyethyl acrylate charge containing 4.0 weight percent of *p*-methoxyphenol (as polymerization inhibitor), and 13.0 weight percent of salicylic acid, equal to 5.2 percent of the initial acrylic acid charge by weight. The transferred mixture would then be held for 2.8 hours at 42 °C under a dynamic vacuum of 0.60 kPa to strip excess ethylene oxide. During the strip phase air would be introduced prior to the reboiler at a rate of 1.7*10⁻³ liters per minute per liter of distillation vessel volume. Next the material would be distilled at a vacuum of less than 0.07 kPa with a mixture temperature range of 58 to 73 °C. During the distillation phase a solution of the ammonium salt of N-nitrosophenylhydroxylamine, water and hydroxyethyl acrylate with a relative ratio of 1 : 2.8 : 6.2 would be introduced to the distillation vessel at a rate of 9.3*10⁻⁵ liters per hour per liter of distillation vessel volume. Also during the distillation phase, air at a rate of 1.7*10⁻³ liters per minute per liter of distillation vessel volume would be added prior to the reboiler, and air would be added at a rate of 4.3*10⁻⁴ liters per minute per liter of distillation vessel volume to the contact condenser loop. Furthermore, during the distillation phase, recycled hydroxyethyl acrylate equal to 44 percent of the initial acrylic acid charge by weight would be added to the crude mixture contained in the distillation vessel. Product would be collected in receiver vessels. The use of the ammonium salt of N-nitrosophenylhydroxylamine solution with the ratio of the ammonium salt of N-nitrosophenylhydroxylamine to water to HEA of 1 : 2.8 : 6.2 allows more of the ammonium salt of N-nitrosophenylhydroxylamine to be added per unit of water compared to a saturated aqueous solution containing the ammonium salt of N-nitrosophenylhydroxylamine, which is approximately 10 percent. The addition of more of the ammonium salt of N-nitrosophenylhydroxylamine per unit of water should allow for better vapor phase inhibition of polymerization without increasing water contamination in the final distilled product relative to the use of a saturated aqueous solution containing the ammonium salt of N-nitrosophenylhydroxylamine.

### Example 5

A procedure was carried out in the same way as of Example 2 except after the transfer was complete from the reactor to the distillation apparatus, the washing of the inside of the reactor was performed in the following manner. In order to remove the residual crude hydroxypropyl acrylate from the reactor, acetic acid was added to the vessel in an amount of 39 percent volume of the inside of the reactor, and the contents were agitated for 15 minutes and then drained. The used acetic acid was stored for future cleaning of the reactor and distillation apparatus. Next water was added to the vessel in an amount of 99 percent volume of the inside of the reactor. The reactor contents were then sparged with air at a rate of 7.9*10⁻² liters per minute per liter of reactor vessel volume and with nitrogen at a rate of 0.12 liters per minute per liter of reactor vessel volume and agitated for 2 hours and then drained. The water extraction process was repeated twice. Next the reactor was dried by pressuring the vessel with nitrogen and air to 207 kPa and then venting the vessel to 34 kPa. The drying procedure was repeated 10 times. After the distillation was complete, the washing of the inside of the distillation vessel was performed in the following manner. In order to remove the residual crude hydroxypropyl acrylate, acetic acid was added to the vessel in an amount of 7 percent volume of the inside of the distillation vessel, and the contents were circulated for 1 hour. Thereafter the washing of the inside of the distillation vessel used water in an amount of 90 percent volume of the inside of the distillation vessel, and the contents were circulated for 30 minutes and then drained. The water extraction procedure was repeated twice. Next the distillation vessel was dried by applying vacuum to the vessel and then venting to the atmosphere. The drying procedure was repeated twice.

### Comparative Example 5

A procedure was carried out in the same way as of Example 2 except after the transfer was complete from the reactor to the distillation apparatus, the washing of the inside of the reactor was performed in the following manner. In order to remove the residual crude hydroxypropyl acrylate from the reactor, water was added to the vessel in an amount of 99 percent volume of the inside of the reactor. The reactor contents were then sparged with air at a rate of 7.9*10⁻² liters per minute per liter of reactor vessel volume and with nitrogen at a rate of 0.12 liters per minute per liter of reactor vessel volume and agitated for 2 hours and then drained. The extraction procedure was repeated twice. Next the reactor was dried by pressuring the vessel with nitrogen and air to 207 kPa and then venting the vessel to 34 kPa. The drying procedure was repeated 10 times. After the distillation was complete, the washing of the inside of the distillation vessel was performed in the following manner. In order to remove the residual crude hydroxypropyl acrylate, acetic acid was added to the vessel in an amount of 7 percent volume of the inside of the distillation vessel, and the contents were circulated for 1 hour. Thereafter the washing of the inside of the distillation vessel used water in an amount of 90 percent volume of the inside of the distillation vessel, and the contents were circulated for 30 minutes and then drained. The water extraction procedure was repeated twice. Next the distillation vessel was dried by applying vacuum to the vessel and then venting to the atmosphere. The drying procedure was repeated twice.

## Claims

1. A process for producing a high purity hydroxyalkyl (meth)acrylate comprising:
i) feeding (meth)acrylic acid into a reaction vessel;
ii) feeding at least one alkylene oxide into the reaction vessel at a feed rate in the range of 0.12 to 1.23 mole of alkylene oxide per mole of (meth)acrylic acid per hour;
iii) reacting the at least one acrylic acid and at least one alkylene oxide to form a hydroxyalkyl (meth)acrylate; and
iv) purifying the hydroxyalkyl (meth)acrylate via distillation.

2. The process of claim 1 further comprising cleaning the reaction vessel with at least one wash wherein the components of the wash consist essentially and of acetic acid and water.
